Europäisches Patentamt

European Patent Office

Office européen des brevets

(11) Publication number: **0 387 187 B1**

# EUROPEAN PATENT SPECIFICATION

(45) Date of publication of patent specification: **14.12.94**

(51) Int. Cl.⁵: **D21C 9/08**, C12S 3/08, C12R 1/645

(21) Application number: **90810093.6**

(22) Date of filing: **09.02.90**

(54) **Process for reducing the pitch content in pulpwood.**

(30) Priority: **13.02.89 US 310814**

(43) Date of publication of application:
**12.09.90 Bulletin 90/37**

(45) Publication of the grant of the patent:
**14.12.94 Bulletin 94/50**

(84) Designated Contracting States:
**AT BE CH DE ES FR GB IT LI NL SE**

(56) References cited:
CH-A- 667 673
GB-A- 1 189 604

**M.J. Wingfield et al "Ceratocystis and Ophiostoma Taxonomy, Ecology and Pathogenicity"; Chapter 16 by K.A. Seifert "Sapstain of Commercial Lumber by Species of Ophiostoma and Ceratocystis" 1993, ABS Press, St. Paul, pages 141-151.**

**Svensk Papperstidning, Vol. 73, Nr. 16, August 1970, Sweden; A. Assarsson et al "Outside Chip Storage (OCS)" pages 493-501**

(73) Proprietor: **SANDOZ AG**
**Lichtstrasse 35**

**CH-4002 Basel (CH)**

(84) Designated Contracting States:
**BE CH ES FR GB IT LI NL SE AT**

(73) Proprietor: **SANDOZ-PATENT-GMBH**
**Humboldtstrasse 3**
**D-79539 Lörrach (DE)**

(84) Designated Contracting States:
**DE**

(72) Inventor: **Blanchette, Robert A.**
**975 West County Road I**
**Shoreview, Minnesota 55126 (US)**
Inventor: **Farrell, Roberta Lee**
**177 Hobart Street**
**Danvers, Massachusetts 01923 (US)**
Inventor: **Hadar, Yitzak**
**2301 Stears Hill Road**
**Waltham, Massachusetts 02154 (US)**
Inventor: **Merritt, Johnnie E.**
**Route 3,**
**Box 2056**
**Ashland, Virginia 23005 (US)**
Inventor: **Snyder, Robert A.**
**105 Riverside Drive**
**Ashland, Virginia 23005 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Wendler, Philip A.**
**4 Worcester Street**
**Belmont, Massachusetts 02178 (US)**

# EP 0 387 187 B1

## Description

The present invention relates to paper making industry and more particularly provides a process and a composition for reducing the resin content of wood used for making mechanical or chemical woodpulp.

Resin, commonly called pitch, is a product naturally found in wood of a large number of species and is of no value for paper production. The presence of resin in pulp is generally considered to be a negative factor which may adversely affect the quality of the pulp and the paper prepared therefrom. Additionally, in pulping processes, resin can form a deposit in the ducts and on the inner surfaces of the apparatuses which may clog upon normal operation. This is a serious problem for maintenance and productivity since repeated and lengthy cleanings are necessary, during which time the production has to be stopped.

In paper making industry, wood intended for pulp making, typically in the form of wood chips or sawdust, is very commonly stored in open air for several days and even some weeks before entering the pulping operation.

In one aspect, outdoor storage seems to facilitate the gradual breakdown of the resinous component. This has been attributed in part to some chemical reactions e.g. hydrolysis of the glyceride constituent and oxidation of the saturated components which occur in moisturized environment and, in part, to microbial action. However, components of resin, such as waxes are only partially or not at all degraded by such oxidative or hydrolytic mechanisms and generally persist in pulp.

In another aspect, although outdoor storage is economically advantageous, it may adversely affect the quality of pulp since, during this period of time, bacterial or fungal infections may develop and lead to a substantial degradation of wood chips.

Throughout the year, but more particularly in summer, wood chips are often infected with a type of fungi which usually appear as a dark stain on the wood. Such a staining penetrates the wood in depth and reflects the invasion of the ray parenchyma cells and resin ducts by the mycelium (hyphae) of the fungus. In the beginning, the infection in a wood pile is generally localized but may spread, particularly in warm weather. Since the staining of wood may persist within the pulp and paper made therefrom, dark penetrating staining has up until now been considered a serious defect and it has been common practice to remove the infected portions of a pile before spreading.

GB-A-1 189 604 describes a process for removing resin constituents from wood chips which comprises applying a fungus which, while it degrades resinous components, it will simultaneously impede the development of cellulose degrading (wood decay) fungi. The article "Outside Chip Storage" by Assarsson et al.,pp. 493-501 in Svensk Papperstidning Vol. 73, Nr. 16, 1970, discusses the effect of microbiological and chemical processes in the wood and lists wood discoloration, caused by staining fungi, among the disadvantages of uncontrolled chip storage. CH-A-667 673 describes a process for the production of lignolytic solutions by cultivation of basidiomycetes and suggests the addition of stabilizers to such solutions.

Surprisingly, it has now been found that penetrating fungi herein indicated, e.g. penetrating dark (blue) stain fungi, are able to substantially degrade the resin content of wood and have therefore so great a beneficial effect on the quality of wood intended for pulp making that staining now appears as a minor defect. Not only the problems associated with the presence of resin in pulp are reduced but also the strength properties of the paper are improved.

Accordingly the invention provides a process for reducing the resin content of wood which comprises applying an inoculum of a resin-degrading fungus to the wood and maintaining environmental conditions effective for promoting the fungal growth, characterized in that the inoculum is biologically pure and the resin degrading fungus is a wood-penetrating dark or faded stain fungus (hereafter called penetrating fungus).

The invention further provides a composition comprising a fungal material together with a stabilizing agent, characterized in that the fungal material is biologically pure and derived from a resin-degrading, wood-penetrating dark or faded stain fungus.

By "resin" is meant any substance insoluble in water but soluble in organic solvents such as ethanol, methylene chloride, diethyl ether, benzene/alcohol mixture and the like. A large variety of resin exist and include for example, terpenes, diterpene acids, fatty acids, esters, glycerides, waxes and alcohols.

Wood to be treated according to the process of the invention may be hardwood or softwood and includes without limitation, birch, oak, poplar, tupelo, beech and conifers such as pines, cedars, spruces, firs (Douglas firs), yew, cypresses, larches and tamaracks and preferably is pine of any kind.

Suitable forms of wood for use in the process of the invention include debarked or undebarked cut timbers, mechanical pulps of any kind and refined pulpwood, this latter form being preferred. Pulpwood is advantageously maintained in an accumulated mass.

3

Cut timbers may be conveniently inoculated by applying an inoculum of a penetrating fungus on cross-sections or on the trunk which may be advantageously scored to facilitate the infection.

By "mechanical pulp" is meant a pulp which have been subjected to any of the well-known mechanical pulping treatments or to at least one step of such treatments and which, therefore, still contains a relatively high amount of lignin e.g. 60% or more of the original lignin content. One example of such pulps is the pulp resulting from the primary stage of a thermomechanical pulping process.

By "refined pulpwood" is meant any portion of wood obtained as a result of mechanical or shearing forces applied to a timber to obtain a multiplicity of surface areas, small pieces or particles suitable for use in the primary stage of any pulp making process. Wood chips and sawdust represent two common refined pulpwoods.

It is preferred that the wood to be treated be a fresh wood i.e. timbers newly cut or wood chips newly prepared preferably from fresh timbers. However, aged wood may be also used, if desired.

Microscopic analysis of wood infected by a penetrating fungus indicates that such a fungus invades, upon growth, the ray parenchyma cells of both softwood and hardwood ad the resin ducts of softwood. Typically, at least 50% of the parenchyma cells ad ducts of an infected piece of wood are invaded. A penetrating fungus may appear as a deep coloured stain on wood that cannot be readily planed off. Under appropriate growth conditions, such a penetrating stain fungus is characterized by a staining of at least 6 mm below the inoculated surface of wood, in contrast to the known surface-growing stain fungi. Further description of the whole class of penetrating fungi is provided in Boyce, Forest pathology, 3rd edition, 1961, McGraw-Hill Book Company.

Wood-penetrating stain fungi include those which appear on wood as a dark stain e.g. a black, dark blue and dark grey stain, sometimes tinted, and mutants and variants of dark stain fungi which are faded stain fungi. Dark stain fungi are preferred for use in the process of the invention.

Penetrating fungi are typically found in the group of Ascomycetes or Deuteromycetes, more particularly in a wide variety of genera which comprises the genera classified in the sub-class Ophiostomatales as well as the genera including the imperfect states associated to Ophiostomatales. Examples of such genera include without limitation Ceratocystis, Ceratocystiopsis, Graphium, Leptographium, Ophiostoma, Phialocephala and Sporothrix as defined with reference to the generic concepts stated in Harrington T.C., New combinations in Ophiostoma or Ceratocystis species with Leptographium anamorphs, Mycotaxon, 1987,28: 39-43 and in Leptographium root diseases conifers, Harrington T.C. & Cobb F.W., 1988, pages 1-39, APS press, St. Paul, Minnesota as well as Rhinocladiella and Hyalodendron as defined with reference to Hawksworth et al., Ainsworth and Bisby's dictionary of fungi, 1983, 7th edition, Commonwealth mycological institute, Kew, Surrey, England. Other Examples of genera (not classified as Ophiostomatales) in which penetrating fungi may be found on a limited species basis include Alternaria, Cadophora, Chloridium, Diplodia, Dactylella, Fusarium, Hormodendron, Hormonema, Phialophora, Sphaeropsis, Trichosporium, Codinaea and Valsa as defined with reference to Hawksworth et al. (supra). Preferred fungi are found in the genera Chloridium, Dactylella, Phialophora and Valsa as well as in the genera classified as Ophiostomatales or including the imperfect states associated with Ophiostomatales, these latter genera being particularly preferred. More preferably, the fungi are found in the genera Ceratocystis and Ophiostoma, this latter being mostly preferred.

Hereinafter is provided a list of species to illustrate the diversity of the group of the penetrating fungi. In this list O. indicates Ophiostoma, C. indicates Ceratocystis and L. indicates Leptographium. The same species may be represented by several strains, indicating different sources or locations at which the same species have been identified, although varietal differences depending on location may also be encountered. The list also indicates that a fungal species may naturally infect a wide variety of wood species or several wood genera. Both terms "species and genus", whether they are applied to wood or fungi, are used herein according to their meaning as defined by the general taxonomic rules.

| Strain | Species | Wood from which the strain was isolated | Location |
|--------|---------|------------------------------------------|----------|
| 5 | L. procerum | Ponderosa pine | |
| 6 | L. abietinum | Ponderosa pine | |
| 10 | L. procerum | Austrian pine | |
| 14 | L. sp. | Maritime pine | |
| 16 | L. procerum | Monterey pine | |
| 19 | L. procerum | White pine | New Zealand |
| 20 | L. procerum | White pine | Ontario |
| 21 | L. truncatum | | New Zealand |
| 22 | L. sp. | Loblolly pine | |
| 25 | L. procerum | White pine | Yugoslavia |
| 25 | L. procerum | Fraser Fir | |
| 27 | L. procerum | Jack pine | Minnesota |
| 28 | L. truncatum | Loblolly pine | |
| 32 | L. abietinum | Engelman spruce | Victoria Canada |
| 34 | L. procerum | Weevil hylobius | Minnesota |
| 37 | L. serpens | Loblolly pine | |
| 39 | L. penicillatum | Ponderosa pine | |

| Strain | Species | Wood from which the strain was isolated | Location |
|---|---|---|---|
| 41 | L. serpens | White pine | |
| 45 | L. terebrantis | Scotch pine | Minnesota |
| 47 | L. terebrantis | Bark Beetle | California |
| 48 | L. lundbergii ATCC 2235 | | |
| 54 | L. wageneri | Douglas Fir | California |
| 56 | L. procerum | White pine | Illinois |
| 57 | L. procerum | White pine | Pennsylvania |
| 58 | L. procerum | Red pine | Minnesota |
| 59 | L. procerum | Grand fir | Washington |
| 60 | L. serpens | Pinus pinaster | South Africa |
| 61 | C. subanulata | Ponderosa pine | |
| 62 | O. microsporum | | |
| 64 | O. piliferum | | |
| 65 | O. abiocarpum | | |
| 68 | C. fraxinopennsylvanica | | |
| 69 | O. piceae | | |
| 70 | C. coerulescens | | |
| 71 | C. adiposa | | |
| 72 | O. tremulo-aureum | | Minnesota |
| 73 | O. huntii | | |
| 74 | O. gossypinum | | |
| 76 | O. piliferum | Ponderosa pine | |
| 77 | O. populinum | | |
| 79 | C. virescens | | |
| 83 | O. minus | Red pine | Minnesota |
| 84 | O. ips | Red pine | Minnesota |
| 89 | C. eucastaneae | | |
| 91 | C. californica | | |
| 93 | O. minutum | | |
| 96 | O. galeiformis | | |
| 97 | C. tenella | | |
| 101 | O. stenoceras | | |
| 106 | O. brevicollis | | |
| 107 | C. ponderosae | | |
| 108 | O. pluriannulatum | | |

| Strain | Species | Wood from which the strain was isolated | Location |
|--------|---------|------------------------------------------|----------|
| 110 | O. distortum | | |
| 113 | O. olivaceum | | |
| 114 | O. robustum | | |
| 115 | O. dryocoetidis | | |
| 116 | C. olivaceapini | | |
| 118 | C. ambrosia | | |
| 120 | O. populinum | | |
| 123 | L. penicillatum | | Vermont |
| 124 | Graphium sp. | Red pine | |
| 125 | O. ips | Scots pine | Minnesota |
| 126 | O. clavigerum | Lodgepole pine | Wyoming |
| 132 | C. eucastaneae | Chestnut canker | |
| 133 | O. olivaceum | | |
| 135 | O. adjuncti | | |
| 139 | O. aureum | | |
| 141 | O. europhioides | | |
| 144 | C. tenella | | |
| 146 | C. denticulata | | |
| 150 | C. allantospora | Red pine | Wisconsin |
| 153 | O. piceae | Red oak | Minnesota |
| 154 | Chloridium virescens var. chlamydosporum | Jack pine | Wisconsin |
| 155 | C. sp. | Jack pine | Wisconsin |
| 157 | Chloridium sp. | Jack pine | Wisconsin |
| 158 | Dactylella sp. | Jack pine | Wisconsin |
| 159 | O. minus | Red pine | Wisconsin |
| 160 | O. tetropi | Red pine | Wisconsin |
| 162 | Ceratocystiopsis sp. | Jack pine | Wisconsin |
| 165 | Dactylella sp. | White pine weevil | Wisconsin |
| 166 | Phialocephala bactrospora ATCC 44606 | | |
| 167 | L. sp. ATCC 12867 | | |
| 168 | Phialocephala dimorphospora ATCC 24087 | | |

| Strain | Species | Wood from which the strain was isolated | Location |
|---|---|---|---|
| 169 | Leptographium pyrinum | ATCC 34943 | |
| 171 | Phialocephala fusca | Maple | |
| 173 | O. minus | M. scuttellatus | Wisconsin |
| 175 | Codinaea sp. | Monochamus carolinensis | |
| 178 | Dactylella sp. | M. carolinensis | |
| 181 | O. penicillatum | | NSW, Australia |
| 186 | O. ulmi | American elm | St. Paul, Minnesota |
| 189 | Diplodia pinea | Jack pine | BRF, Wisconsin |
| 190 | Diplodia pinea | Red pine | |
| 191 | O. ips | Austrian pine | Minnesota |
| 192 | O. minus | Austrian pine | Minnesota |

Among the species cited in the above list those numbered 61, 62, 64, 65, 68-74, 76, 77, 79, 83, 84, 89, 91, 93, 94, 96-120, 124-150 153, 154, 157, 158, 162, 165, 166, 171, 175, 181, 189 and 190 are of particular interest.

A faded stain fungus may be isolated as a variant or mutant from a parental strain appearing as a dark stain. In an old culture, e.g. 5-9 day old culture, of a dark stain fungus, spontaneous faded variants or mutants typically arise and appear as light grey to medium grey spots after plating of culture e.g. as done for serial dilution. They may be picked up and grown as individual isolates. Alternatively, they may be also produced through mutation experiments. Preferred faded stain fungi exhibit a good growth vitality e.g. similar to that of the dark stain fungi.

In nature, penetrating fungi e.g. blue stain fungi are usually heterokaryotic. Indeed, in the course of a culture, the nuclei segregate in cells in various combinations which change the characteristics of the strain. However, homokaryotic strain may be selected and may be of preferred use in the process of the invention because the characteristics of such a strain are stable. Selection may be achieved as follows: Ascospores of a heterokaryotic strain are, by definition, homokaryotic. Therefore, spores may be recovered and individually separated e.g. plated on a solid growth medium at a dilution which allows separate growth. Then the resulting strains are tested for their homokaryotic nature. Alternatively, two heterokaryotic strains may be crossed together and the lineage is analysed as described above.

Advantageously, a penetrating fungus for use in the process of the invention is characterised by at least one of the following properties:

a) it does not substantially degrade the cellulosic content of wood,
b) it is not pathogenic for the living matter,
c) it is able to grow on different wood species,
d) it is able to grow on different wood genera,
e) it is able to strongly or quickly grow in a competitive non-sterile environment (i.e. its growth is not substantially inhibited by the presence of other microorganisms),
f) it inhibits the growth of other microorganisms.

Also advantageously, the fungal strain chosen for use in the process of the invention is that which naturally infects the wood species to be treated.

The inoculum is a composition which comprises a fungal material of a penetrating fungus e.g. a fungal culture or a fungal preparation derived from a fungal culture. The inoculum is biologically pure i.e. it is substantially free of microorganisms other than penetrating fungi or derived from a biologically pure culture. Biologically pure cultures of a desired fungus may be obtained in liquid or solid form by methods well-

8

known in the art. Cultures carried out on a solid substrate may be of some particular interest since the resulting cells are resistant to desiccation. Typically, a fungal culture is a mixture of at least two different fungal forms i.e. hyphae and spores, each form being able to successively predominate as the culture grows. A certain type of spores appears as yeast-like cells, more particularly in liquid culture. A fungal preparation, e.g. a spore suspension, may be prepared from a fungal culture by standard techniques. For use in the process of the invention, the inoculum comprises a fungal culture or a fungal preparation which comprises at least 50%, more preferably at least 80%, most preferably at least 90% of spores, those being preferably yeast-like cells.

The inoculum may be in liquid or dry form e.g. lyophilized. When the inoculum is stored in dry form before use, it may then be applied as such or diluted. When a dry inoculum is applied to the wood, then it is advantageous to separately moisten the wood. In the process of the invention, it is particularly preferred to keep the inoculum frozen, at at least -10°C, preferably at at least -15°C, suitably at about -20°C before use.

The inoculum may also comprise additives such as preservatives or stabilizing agents. Examples of preservatives or stabilizing agents include silicon dioxide, skim milk, polyethylene glycol, polypropylene glycol and sugars such as fructose, glucose and sucrose.

The inoculum may contain one or several fungal species. Alternatively, several inocula containing a single species may be applied to the wood concomitently or subsequently.

The inoculum may be intentionally applied to the wood in a variety of manners. Typically, the inoculum is applied in a systematic or methodical manner. For example, the inoculum is distributed at intervals into the mass of wood e.g., a pile of refined pulpwood, or on the outer surface of a cut timber, preferably at regular intervals. More preferably, the inoculum is diffused in a homogeneous manner, i.e. substantially throughout the mass of wood. However, it is not necessary that each individual wood chip, sawdust particle and the like be inoculated. As little as 10 % and even less but preferably about at least 20 % more preferably about 50 %, most preferably about 85 % of the individual pieces shall be inoculated since the uninoculated pieces are accumulated in contact with the inoculated pieces. Upon growth, the infection will spread very easily.

A thorough and uniform inoculation of a mass of wood is generally reflected by the fact that the fungus grows substantially throughout the mass. However, for unknown reasons, it may happen that some part of the mass, particularly the outer layer of a pile of refined wood pulp, shows little growth compared to the rest of the mass, or no growth at all, although it has been inoculated.

In one preferred embodiment, the inoculum is sprayed onto wood chips or sawdust as they are discharged from the refining operation but before being accumulated into piles. For example, a wood chipping apparatus is generally provided with conveyor means which receive the newly prepared chips and convey them to the accumulating pile. A spray applicator containing the inoculum preparation may be conveniently adapted to the conveyor, preferably at the junction with the chipper when the chips are airborne e.g. free falling or tumbling, or at the very end of the conveyor so that chips are sprayed when falling from the conveyor.

Alternatively, the inoculum may be applied to the wood chip pile in the course of its accumulation by more or less continuous spraying over the accumulating pile.

After inoculation, the accumulated mass is maintained under conditions which will promote the growth of the fungus substantially throughout the mass. Given the fact that the invention will in most cases be likely to be practised in open air and the mass therefore subjected to a wide variety of weather conditions, the maintenance of any given set of ideal conditions throughout the entire treatment period is usually too difficult to achieve and is often unnecessary in practice. It is generally sufficient that the mass be substantially maintained at a temperature at which the fungus grows while avoiding higher temperatures at which the fungus dies. Accordingly, a penetrating stain will be advantageously selected among those adapted to the local temperature conditions. While many fungi may exhibit some reasonable growth at or below 0°C it will generally be more suitable to attain a temperature of at least 10°C, such as a temperature of from 10°C to 40°C, more preferably of from 15°C to 33°C, most preferably of from 22°C to 28°C. In the course of the year, the use of different fungi, each adapted to the seasonal temperature is of course within the scope of the invention.

In warm weather conditions, it is not necessary to influence the environmental temperature and the inoculated mass may be left to stand in open air without special maintenance. In cold weather conditions, it is desirable to provide the inoculated mass with means for maintaining a suitable temperature. This may be a heat-retaining covering placed over or on the inoculated mass such as a large plastic sheet or a concrete "igloo" or any similar structure which can be internally heated and emit radiant heat. Alternatively, the ground base on which is placed the inoculated mass may be provided with heating pipes or a plurality of openings for releasing warm air or steam. When providing heating means, it would also be desirable to control the moisture conditions to avoid an excessive dryness. In view of this, means for venting the heat or steam would be adequate.

The period of time during which the infection is left to develop in a refined pulpwood may vary considerably upon a number of factors including the desired extent of resin removal, the temperature and moisture conditions, the original microbial conditions of wood, the extent of inoculation and the particular fungus which is used. However, satisfactory results may generally be obtained after a period of time extending from 4 to 45 days, preferably from 7 to 35 days. Under preferred conditions, very effective results e.g. a pitch reduction of about 20 % or more may be obtained 5 to 25 days after the inoculation.

Treatment of cut timbers will usually be somewhat longer than that of refined pulpwood and may extend for 2 months and even more.

Wood e.g. wood chip pile treated according to the process of the invention and which have been incubated for less than 6 weeks substantially differ from an untreated pile in that the amount of chips in the treated pile which show a visible growth of a deep penetrating stain fungus is at least 25%, preferably 35%, more preferably 50% of the total amount of chips.

The wood treated according to the process of the invention is suitable for use in any conventional pulping process such as the mechanical, thermomechanical, chemimechanical, chemithermomechanical and chemical pulping treatments. These treatments are usually followed by a bleaching or brightening-effect treatment of the pulp and if necessary, minor adjustments to the bleaching step may be made to eliminate a residual staining of the final product.

In the following examples, unless otherwise indicated, resin is quantified according to standard TAPPI procedure T 204 om-88 which is slightly modified as follows.

Wood chips are splintered using pruning shears to a width of about 1 cm. The resulting splinters are oven dried overnight at about 60°C and then ground into sawdust using a Thomas-Wiley Intermediate Mill with a screen having 3.1 apertures per $cm^2$(20-mesh). Four grams of the dried sawdust are mixed with approximately 20 ml methylene chloride (dichloromethane-DCM) and the resulting mixture is agitated overnight at room temperature to remove extractable components from the sawdust. The liquid is then pipetted from the mixture and filtered through a 0.45 $\mu$m (micron) organic filter. The recovered liquid is then evaporated overnight at room temperature. The residues are placed in an oven at about 60°C for 30 minutes to further remove methylene chloride. Pitch content is obtained by weighing the residue after methylene chloride removal and expressing the result as milligram of pitch content per gram of substrate which has been extracted with methylene chloride.

In the following examples, unless otherwise specified, the wood chips employed are made from southern yellow pine harvested in the state of Virginia, U.S.A., using a mixture of two-thirds freshly cut timber and one-third cut timber which has been field-aged for about three months.

In the following examples, F1 is the species Ophiostoma piceae, G1 is the species Ceratocystis adiposa, I1 is the species Ophiostoma piliferum, (first isolate), C1 is also Ophiostoma piliferum (second isolate) and E1 is a Graphium sp., each being isolated from selected samples of the above-identified southern yellow pine wood chips which were found at a pulp and paper company plant in Virginia, and produced from and used in the following examples as biologically pure cultures.

In the following examples, the inoculated chips and control chips are contained in sealed plastic bags during the periods of treatments. In such experiments, chips are frozen at -20°C until use.

In the following examples, two kind of control samples are indicated, in experiments carried out with non-sterilized chips, namely a frozen control and a true control. The frozen control represents woodchips at the zero time point of the experiment. The true control represents woodchips in which the natural microbial mass was allowed to grow during the course of the experiment. The decrease in pitch content of the true control reflects the degradation which naturally occurs due to the microorganisms originally present within the chips.

Accordingly, the invention is further illustrated as follows:

Example 1

200 g samples of non-sterile winter wood chips are inoculated with a culture of F1, G1 or I1 prepared and harvested on solid malt agar plates. The inoculated chips are then incubated at room temperature for 3 weeks. The pitch contents compared with that of a frozen control are given below.

| Sample | Pitch content mg Pitch / g Substrate |
|---|---|
| Frozen control | 32.3 |
| F1 | 21.9 |
| G1 | 19.9 |
| I1 | 13.7 |

Example 2

300 g samples of summer wood chips (October pile) are sterilized and then inocubated with a spore suspension of F1 (1.5 $10^7$ spores), G1 (1.$10^7$ spores), I1 (1.2 $10^7$ spores) or G1 and I1. Chips are wet with 10 ml water/100 g wood chips to facilitate the growth of fungus. Chip samples are incubated under these conditions for 11 or 27 days at room temperature and pitch content is measured. The results compared to a frozen and unfrozen controls are given below.

| Sample | Pitch content mg pitch/g substrate | |
|---|---|---|
| | (11 days) | (27 days) |
| Frozen control | 28.2 | 32.3 |
| Control | 29.4 | 24.7 |
| F1 | 23.8 | 23.6 |
| G1 | 24.9 | 16.4 |
| I1 | 20.9 | 19.8 |
| G1 and I1 | 21.8 | 20.1 |

Example 3

I1 is grown in a rotating (200 RPM) 2 liter flask containing 500 ml liquid basal malt extract at 25°C. The inoculum consists of 5mm agar plugs containing sporulating fungi. In the course of the incubation, the rate of spore production I1 is determined as reported below.

| Incubation | Spore count (spores/ml) |
|---|---|
| 48 hrs | 8.7 x $10^7$ |
| 72 hrs | 3 x $10^8$ |
| 96 hrs | 5 x $10^8$ |

After the 48th hour of incubation, spore viability was determined using a dilution plate assay and found to be 11 x $10^7$ colony forming units/ml.

A 48 hour liquid culture is centrifuged and the pellet is resuspended in a small quantity of water to provide for a concentrated suspension to which is added 10 % dry skimmed milk as stabilizer. Such a suspension constitutes a suitable inoculum to be used in the process of the invention.

Example 4

An outdoor experiment using two wood chip piles of 12 ton each is conducted at a site in southern Virginia, U.S.A., in early December.

The wood to be inoculated is made of approximately 60 % chips obtained from fresh-cut wood and 40 % chips from logs stored in open air for at least 3 months. Wood chips have an average pitch content of 34 mg/g.

A concentrated inoculum of I1 is diluted with water to contain approximately 3 x $10^{12}$ spores in 10 l distilled water, thus providing approximately 2 x $10^8$ spores per kilogram of chips. 10 l inoculum is intermittently sprayed on loads of chips used in forming pile II and onto pile II after the pile is formed. Piles I (not inoculated) and II are tarped with a clear plastic. Pile I is kept unheated and temperature sensors indicate temperatures in this pile in the range of -5°C to 10°C. Pile II is heated by supplying warm air beneath the pile to obtain a temperature of 20-25°C in most sections of the pile. The forced air heater is within a structure with cider block sides and wire mesh screen top which separates the chips from the heater.

After 15 days of incubation, chip samples are randomly taken from each pile to constitute 2 samples of approximately 135 Kg each which are subsequently treated by a thermomechanical pulping process. The pitch content of the resulting pulp is then measured. The average pitch content of the pulp derived from pile I and pile II is 27 and 21 mg/g, respectively.

Physical properties of the pulp and of the paper obtained therefrom including burst factor, tear factor, breaking length and stretch are investigated according to standard procedures. It is indicated that the material (pulp or paper) derived form pile II is of better quality than the material derived from pile I or from untreated fresh wood chips.

Example 5

October chips are stored for 1 week at 4°C before inoculation with a fungus. 400 g samples are inoculated with the fungi indicated below with a total of about $10^7$ spores involved in each inoculation. Water is added at 15 ml/100 g chips to some samples to determine the influence of moisture conditions on pitch degradation. Results are given below.

| Sample | Pitch content mg Pitch/g substrate | |
|---|---|---|
| | (17 days) | (25 days) |
| Frozen control | - | 35.4 |
| Frozen control + water | 33.8 | 29.8 |
| F1 | 23.0 | 19.4 |
| F1 + water | 22.7 | 21.4 |
| G1 | 20.7 | 15.8 |
| G1 + water | 34.2 | 27.6 |
| I1 | 23.2 | 20.4 |
| I1 + water | 25.7 | 17.8 |
| I1 + G1 | 16.7 | 15.7 |
| I1 + G1 + water | 30.4 | 18.9 |

The weight of the chips before and after 25 day incubation is also determined. There is little water loss during incubation.

The greatest pitch reduction in the shorter time period in these samples is observed when I1 and G1 are coinoculated.

Example 6

October chip samples are inoculated with different inocula as reported below. After 21 day incubation the pitch content is measured.

| Strain | Spores/ml | Pitch content (mg/g substrate) |
|---|---|---|
| Frozen control | | 24.4 |
| I1 | $6x10^4$ | 17.8 |
| I1 | $6x10^5$ | 14.7 |
| I1 | $6x10^6$ | 15.1 |
| I1 | $6x10^7$ | 16.7 |
| I1 | $6x10^8$ | 21.8 |
| I1 | $1x10^9$ | 19.3 |

These results indicate that the inoculum concentration may influence the pitch degradation process.

Example 7

65 g samples of splintered chips (autumn pile, stored frozen) each mixed with 5 ml water are sterilized, cooled at room temperature and inoculated with the fungi indicated below. The samples are maintained for 21 days at room temperature. Results compared to controls are given below.

| Strain | Spores/ml | Pitch content (mg/g substrate) |
|---|---|---|
| Frozen control | | 34.4 |
| Room temp. control | | 32.7 |
| I1 | $9.6 \times 10^7$ | 20.9 |
| G1 | $1 \times 10^7$ | 26.7 |
| F1 | $5 \times 10^7$ | 23.9 |
| C1 | $1.5 \times 10^7$ | 21.9 |
| E1 | $8.6 \times 10^7$ | 24.9 |

Example 8

An I1 culture is grown for 6 days in a standard liquid malt extract medium. Then the culture is centrifuged, and the pellet resuspended in fresh malt extract resulting in a 25-fold concentration. Spore viability is immediately assayed and also assayed after storage for 2 weeks at various temperatures by dilution plate analysis on malt extract agar. Results are to be seen below.

| Storage conditions | Colony forming units/ml |
|---|---|
| no storage | $1 \times 10^{10}$ |
| -20 °C | $4 \times 10^9$ |
| -20 °C | $3 \times 10^9$ |
| 4 °C | $6 \times 10^8$ |
| 25 °C | less than $10^3$ |
| 37 °C | less than $10^3$ |

In a second experiment, a 5 day old culture of I1 is centrifuged, frozen at -20 °C, and lyophilized. Spore viability was assayed after storage for 1 week at various temperatures by dilution plate analysis on malt extract agar. Results are to be seen below.

| Storage conditions | Colony forming units/ml |
|---|---|
| no storage | $5 \times 10^9$ |
| -20 °C as frozen pellet | $3 \times 10^8$ |
| -20 °C, lyophilized | $7 \times 10^7$ |
| 4 °C, lyophilized | $8 \times 10^7$ |
| 25 °C, lyophilized | $6 \times 10^7$ |

Liquid I1 cultures are centrifuged and resuspended in a minimum volume of skim milk. Spores are stored for two weeks and results are indicated below.

| Storage conditions | Colony forming units/ml |
|---|---|
| no storage | $3 \times 10^9$ |
| -20 °C in malt extract | $2 \times 10^8$ |
| -20 °C as frozen pellet | $3 \times 10^8$ |
| -20 °C in skim milk | $7 \times 10^9$ |
| -20 °C in skim milk and lyophilized | $2 \times 10^9$ |

13

Thus, spores may be stored either frozen at -20°C or lyophilized without loss viability.

Example 9 - Fermentation trial of fungus I1

A 10 l fermentation I1 is conducted in a 20l Chemap fermentor. The medium consists of 20 g malt extract (Difco) and 2 g yeast extract per liter. The pH of the medium after autoclaving is 5.9. The inoculum consists of 100 ml of growth medium containing $3 \times 10^8$ spores/ml. Fermentation is conducted at a temperature of 25.1°C, with agitation at 600 rpm, and aeration at 9.4l/min. Foaming is controlled with 20% antifoam B emulsion (Sigma, diluted to 20% strength). pH, dissolved $O_2$, and temperature are measured during operation. Samples are removed periodically for subsequent analysis of cell number.

| Hours fermentation | Spores/ml | pH | $O_2$* | Temperature |
|---|---|---|---|---|
| 0 | $1.7 \times 10^6$ | 5.8 | 54 | 25.1 |
| 4.5 | $4.4 \times 10^6$ | 5.9 | 54 | 25.1 |
| 9 | | | 50 | |
| 11 | | 5.8 | | |
| 14 | | 5.2 | 38 | |
| 19.0 | $9.5 \times 10^6$ | 4.6 | 34 | 25.1 |
| 23.5 | $3.2 \times 10^7$ | 4.4 | 34 | 25.1 |
| 28.5 | $7.2 \times 10^7$ | 4.8 | 34 | 25.1 |
| 32 | | 5.6 | | |
| 43.5 | $2.3 \times 10^8$ | 4.6 | 34 | 25.1 |
| 51 | $2.5 \times 10^8$ | 4.3 | 34 | 25.1 |
| 70 | $3.5 \times 10^8$ | 4.2 | 36 | 25.1 |

* Oxygen levels reported as % saturation.

Examination of the samples under a microscope reveals a higher percentage of hyphae than observed in shake flasks, especially during the early time points. Not until 44 hours do the yeast-like forms predominate. The increased aeration during the fermentation may have caused the alteration in growth morphology. Either a lower rate of aeration or a larger inoculum may force the fungus into the yeast-like state earlier in the incubation. The yeast-like growth and subsequent sporulation state may be preferred because of higher viability in preservation studies.

Example 10

An outdoor experiment involving two chip piles of 2.5 tons each, is carried out in South Carolina, USA in early August. The woodchips are made from freshly cut southern yellow pine and the piles are constructed on plastic sheets.

The fungal inoculum consists of yeast-like cells of Ophiostoma piliferum TAB 28 (dark blue strain) grown for 5 days in liquid culture (2% malt, 0.2% yeast and 750 ml volume in 2 liter flask) at 25°C and subsequently stored in 10% skim milk at -20°C. The inoculum is sprayed onto the chips as the pile is accumulating so that 1 Kg woodchips is inoculated with $10^8$ viable fungal cells. Only one pile is treated, the other one serving as control.

Four weeks after chip samples are randomly taken from each pile. The pitch content of each sample is measured and the average amount is calculated for each pile. The results are given in the table below.

| Pile | average pitch content |
|---|---|
| Control pile | 2.1% |
| Inoculated pile | 1.6% |

The microbial population of each pile is also investigated. Chips randomly taken from the piles are individually placed on solid culture medium (malt and yeast extract agar) and the microbial population growing therefrom is analysed. Results are to be seen below.

14

EP 0 387 187 B1

| Pile | blue stain | bacteria | other fungi |
|---|---|---|---|
| Control pile | 20%* | 100% | 35% |
| Inoculated pile | 90% | 95% | 30% |

* percentage of the infected chips.

Example 11

A 2 liter Erlenmeyer flask containing 750 ml of 2% malt and 0.2% yeast extract is sterilized, cooled and inoculated with plugs of Ophiostoma piliferum TAB 28 grown on malt yeast agar slants. The pH of the medium after sterilization is 5.9. The culture is agitated at 160 rpms at 25°C for 36 hours and then harvested. Samples are removed periodically for subsequent analysis. Examination under the light microscope reveals primarily a mycelium with long hyphae at 18 hours and by 36 hours yeast-like cells predominate (95% of the culture).

Example 12

100 g of woodchips are inoculated with $10^6$ spores of a faded variant of I1. The chips are incubated for 2 weeks at room temperature. After incubation, the pitch content of the control and the treated sample is 2.3% and 1.9%, respectively.

Example 13

Southern yellow pine wood chips which are about 1-2 weeks old and show a blue staining are individually recovered. Fungal isolates are obtained therefrom, grown up and identified. Approximately $10^8$ spores/ml of each isolate are used to inoculated sterilized wood chips which are then incubated for 2 weeks at room temperature. After incubation, the pitch content is measured. Results are to be seen below.

| Fungal isolate | Species | Pitch content (%) |
|---|---|---|
| No isolate (control) | | 2.0 |
| TAB 19 | Pyrenomycete sp | 1.7 |
| TAB 20 | Pyrenomycete sp | 1.7 |
| TAB 21 | Pyrenomycete sp | 1.6 |
| TAB 23 (faded variant) | O. piliferum | 1.5 |
| TAB 25 | O. piliferum | 1.6 |
| TAB 26 | O. piliferum | 1.4 |
| TAB 27 | O. piliferum | 1.4 |
| TAB 28 | O. piliferum | 1.5 |

**Claims**

1.  A process for reducing the resin content of wood which comprises applying an inoculum of a resin-degrading fungus to the wood and maintaining environmental conditions effective for promoting the fungal growth, characterized in that the inoculum is biologically pure and the resin degrading fungus is a wood-penetrating dark or faded stain fungus (hereafter called penetrating fungus).

2.  A process according to claim 1 in which wood is in the form of refined pulpwood.

3.  A process according to claim 1 or 2 in which the wood-penetrating fungus is a wood-penetrating dark stain fungus.

4.  A process according to any one of the preceding claims in which the penetrating fungus is of a genus selected from the group comprising the genera classified in the sub-class Ophiostomatales, the genera including the imperfect states associated to Ophiostomatales and the genera Alternaria, Cadophora,

15

Chloridium, Diplodia, Dactylella, Fusarium, Hormodendron, Hormonema, Phialophora, Sphaeropsis, Trichosporium, Codinaea and Valsa.

5. A process according to claim 4, in which the penetrating fungus is of a genus selected from the group consisting of the genera Ophiostoma and Ceratocystis.

6. A process according to any one of the preceding claims which comprises applying an inoculum which is a composition comprising a fungal material of a penetrating fungus, selected from a fungal culture and a fungal preparation derived from a culture.

7. A process according to claim 6, in which the fungal material comprises at least 50% spores.

8. A process according to any one of the preceding claims which comprises spraying the inoculum onto the wood.

9. A composition comprising a fungal material together with a stabilizing agent, characterized in that the fungal material is biologically pure and derived from a resin-degrading, wood-penetrating dark or faded stain fungus.

10. A composition according to claim 9, in which the fungal material comprises at least 50% spores.

**Patentansprüche**

1. Verfahren zur Reduzierung des Harzgehalts von Holz durch Anwendung eines Inokulums eines harzabbauenden Pilzes auf das Holz und die Erhaltung von Umweltbedingungen, die für die Förderung des Pilzwachstums wirksam sind, dadurch gekennzeichnet, dass das Inokulum biologisch rein ist und der harzabbauende Pilz ein holzdurchdringender dunkler oder verblasster Fleckenpilz ist (hiernach als durchdringender Pilz bezeichnet).

2. Verfahren nach Anspruch 1, in welchem das Holz in Form von aufgeschlagenem Zellstoffholz vorliegt.

3. Verfahren nach Anspruch 1 oder 2, in welchem der holzdurchdringende Pilz ein holzdurchdringender dunkler Fleckenpilz ist.

4. Verfahren nach einem der vorangehenden Ansprüche, in welchem der durchdringende Pilz zu einer Gattung aus der Gruppe von Gattungen, die in der Unterklasse Ophiostomatales klassifiziert sind, gehört, wobei die Gattungen die imperfekten Formen, die mit den Ophiostomatales assoziiert sind, einschliessen oder aus den Gattungen Alternaria, Cadophora, Chloridium, Diplodia, Dactylella, Fusarium, Hormodendron, Hormonema, Phialophora, Sphaeropsis, Trichosporium, Codinaea und Valsa ausgewählt wird.

5. Verfahren nach Anspruch 4, in welchem der durchdringende Pilz einer Gattung aus der Gruppe bestehend aus Ophiostoma und Ceratocystis angehört.

6. Verfahren nach einem der vorangehenden Ansprüche umfassend die Anwendung eines Inokulums, welches eine Zusammensetzung mit Pilzmaterial von einem durchdringenden Pilz ist, ausgewählt aus einer Pilzkultur und einer Pilzzubereitung, die von einer Kultur abstammt.

7. Verfahren nach Anspruch 6, in welchem das Pilzmaterial mindestens 50% Sporen umfasst.

8. Verfahren nach einem der vorangehenden Ansprüche umfassend das Besprühen von Holz mit dem Inokulum.

9. Zusammensetzung umfassend ein Pilzmaterial zusammen mit einem Stabilisierungsmittel, dadurch gekennzeichnet, dass das Pilzmaterial biologisch rein ist und von einem harzabbauenden, holzdurchdringenden, dunklen oder verblassten Fleckenpilz abstammt.

10. Zusammensetzung nach Anspruch 9, in welchem das Pilzmaterial mindestens 50% Sporen umfasst.

**Revendications**

1. Un procédé pour réduire la teneur en résine du bois, comprenant l'application au bois d'un inoculum d'un champignon dégradant la résine et le maintien de conditions d'environnement efficaces pour faciliter la croissance du champignon, caractérisé en ce que l'inoculum est biologiquement pur et que le champignon dégradant la résine est un champignon de teinte sombre ou atténuée pénétrant le bois (désigné ci-après champignon pénétrant).

2. Un procédé selon la revendication 1, dans lequel le bois est sous forme de pâte de bois raffinée.

3. Un procédé selon la revendication 1 ou 2, dans lequel le champignon pénétrant le bois est un champignon de teinte sombre pénétrant le bois.

4. Un procédé selon l'une quelconque des revendications précédentes, dans lequel le champignon pénétrant est d'un genre choisi dans le groupe comprenant les genres répertoriés dans la sous-classe des Ophiostomatales, les genres comprenant les états imparfaits associés aux Ophiostomatales et les genres Alternaria, Cadophora, Chloridium, Diplodia, Dactylella, Fusarium, Hormodendron, Hormonema, Phialophora, Sphaeropsis, Trichosporium, Codinaea et Valsa.

5. Un procédé selon la revendication 4, dans lequel le champignon pénétrant est d'un genre choisi dans le groupe constitué par les genres Ophiostoma et Ceratocystis.

6. Un procédé selon l'une quelconque des revendications précédentes, comprenant l'application d'un inoculum qui est une composition comprenant une matière fongique d'un champignon pénétrant, choisie parmi une culture fongique et une préparation fongique dérivée d'une culture.

7. Un procédé selon la revendication 6, dans lequel la matière fongique comprend au moins 50% de spores.

8. Un procédé selon l'une quelconque des revendications précédentes, comprenant la pulvérisation de l'inoculum sur le bois.

9. Une composition comprenant une matière fongique en association avec un agent stabilisant, caractérisée en ce que la matière fongique est biologiquement pure et dérive d'un champignon de teinte sombre ou atténuée pénétrant le bois.

10. Une composition selon la revendication 9, dans laquelle la matière fongique comprend au moins 50% de spores.